# EUROPEAN PATENT APPLICATION

(11) **EP 2 431 744 A1**
(43) Date of publication of application: **21.03.2012**
(21) Application number: 10382252.4
(22) Date of filing: 20.09.2010
(51) Int. Cl.: G01N 33/68

(54) **In vitro diagnostic method for infant eye allergy**

(71) Applicant: Bioftalmik, S.L., 48160 Derio (Vizcaya) (ES)
(72) Inventor: SUÁREZ CORTÉS, Tatiana, E-48160, Derio - Vizcaya (ES); MARTÍNEZ FERNÁNDEZ, Ricardo, E-48160, Derio - Vizcaya (ES); ACERA OSA, Arantxa, E-48160, Derio - Vizcaya (ES); SORIA ESPONERA, Javier, E-48160, Madrid (ES)
(74) Representative: ABG Patentes, S.L.

(57) **Abstract**

The invention relates to a diagnostic method for diagnosing infant eye allergy based on determining the levels of markers in a biological sample from a child and on comparing said levels with the levels of said markers in a reference sample, wherein an increase in the level of some markers and/or the modulation of others with respect to the levels in a reference sample is indicative of said child suffering from infant eye allergy.

## Description

### Field of the Invention

The invention is comprised within the field in the area of diagnosing diseases, particularly in the development of a diagnostic method for diagnosing infant eye allergy based on the detection of determined markers in tears.

### Background of the Invention

In recent decades, the prevalence of allergic diseases has increased considerably in developed countries such that 25% of the general population presents some type of allergic disease. Most allergy patients present manifestations in diverse target organs, the allergy condition being frequent on an ocular level, being able to cause conjunctivitis.

Allergic conjunctivitis is an inflammatory disease which directly affects the conjunctiva, which is the primary barrier against environmental, chemical and infectious allergens. It produces itching, lacrimation, redness, photophobia or incapacity to withstand light, feeling of grittiness or "cloudiness" in the eyes, and inflammation of the eyelids. Patients rub their eyes in very different ways (with their fingers, underneath glasses, with their fists, with their entire arm, or by blinking their eyes). Thinking that these are nervous tics, they quite frequently visit the ophthalmologist to study visual acuity, or the neurologist or psychiatrist.

In a study conducted by Marrache et al. (Rev Fr Alergol Immunol Clin 1978;18:151-5) in 5,000 allergic children, 32% of these patients presented an eye condition as the sole manifestation of their allergy. In addition, in some cases the eye allergy component is the most incapacitating. Eye allergy is, therefore, a frequent cause of consultations with allergists, ophthalmologists, pediatricians and primary care physicians, including visits to the neurologist or psychiatrist in cases in which the child's continuous blinking is confused with a nervous tic. Greater knowledge of these pathologies, as well as of the immunological mechanisms and of the underlying genetic predisposition, could lead to a better diagnosis, the application of new therapies and therefore to better control of these diseases.

The epidemiological studies carried out on eye allergy are limited. In most studies, eye allergy is considered in the context of other atopic diseases, mainly rhinitis, globally studying rhinoconjunctivitis.

Each of the different eye allergy manifestations presents a different prevalence, depending on various factors, such as the area in which they are studied.

In a prospective study conducted by Belfoti et al. (Acta Ophthalmol Scan 2000;78:38-40) in 134 patients with allergic conjunctivitis in Sao Paulo, visited by eye allergy specialists at the hospital level, it was concluded that 46% of the patients suffered vernal keratoconjunctivitis, 40% atopic keratoconjunctivitis and 8% perennial allergic conjunctivitis.

Epidemiological studies such as the "International Study for Asthma and Allergies in Childhood" demonstrate that there are huge differences in the prevalence of allergic diseases in children and have suggested that ethnic factors are the determining factors in rhinitis and allergic conjunctivitis regardless of the external environment of the child.

The importance of allergic conjunctivitis resides more in its frequency than in its severity. Thus, allergic conjunctivitis in its different forms of seasonal or perennial presentation is the most frequent form of manifestation of eye allergy corresponding to approximately 66% of all allergic eye diseases observed in large series of visited patients.

Allergic conjunctivitis is most frequently associated with rhinitis. In the epidemiological study Alergológica, conducted in Spain in 1995, it was determined that 50% of the patients with rhinitis allergy presented associated conjunctival symptoms.

Depending on the allergen involved, approximately 90% of the patients that present classic symptoms induced by pollens present allergic conjunctivitis in varying degrees of intensity.

In childhood, eye allergies present in three basic forms: seasonal conjunctivitis, perennial conjunctivitis and the most severe form because of the corneal involvement, vernal keratoconjunctivitis.

Seasonal allergic conjunctivitis (SAC) is a mildly aggressive form and together with the perennial form represents the most frequent form of allergic conjunctivitis, being more than 50% of the cases. It is a form of acute conjunctivitis manifested by outbreaks in relation to the pollen season. The symptoms start to appear in spring and, in certain areas, in fall. The eye symptoms are commonly accompanied by nasal and pharyngeal symptoms, the itching, burning sensation and photophobia standing out. There can be mucosal secretion, conjunctival chemosis, eyelid edema and, more infrequently, corneal involvement. Although the symptoms are bilateral, they are not always symmetrical. This type of conjunctivitis does not threaten sight.

Allergic conjunctivitis lasting for more than 3 weeks is considered perennial (Datt et al., 1986 Trans Ophthalmol Soc UK;105:513-520). Chronic or perennial allergic conjunctivitis (PAC) is the result of a mildly intense and continuous antigenic exposure, being more common in an urban environment. It can have to seasonal exacerbation, generally due to pollen allergy from pollen, but it usually has an annual duration due to allergy to dust, mites, fungi, animal hair and other allergens. Its onset is progressive, being able to be acute or subacute after a bacterial or viral infection or because of a chronic trauma, such as the use of contact lenses. Its evolution is initially intermittent to later become chronic.

Finally, vernal keratoconjunctivitis is a form of an allergic eye reaction specific to childhood, subsiding in adolescence but chronically occurring with very severe flare-ups which can lead to corneal ulcers, the cornea possibly being perforated and losing its transparency.

Allergic eye disease cannot be understood solely as a classic type I hypersensitivity reaction, but as a complex process in which cells and diverse mediators are involved. The following stand out among involved cells:
- Mast cells: They are increased in number in allergic eye diseases. Their distribution is also different, since they tend to be distributed towards the epithelium, as well as be situated in the conjunctival stroma. This finding could explain the availability of these cells for reacting rapidly in the presence of allergens.
- T-cells: They are present in the normal lymphoid tissue of the conjunctiva, but their number, phenotype and distribution are also altered in patients with allergic disease. In vernal keratoconjunctivitis, there is an increased CD4/CD8 ratio; the clones also belong more to the Th2 type. Chronic allergic diseases show signs of CD4+ cell activation.
- Eosinophils: They are the effector cells in the allergic reaction, there being an increase in conjunctiva and tears. In perennial conjunctivitis, the eosinophil count is lower in baseline conditions but increases in late allergy reactions. It is interesting that the reaction of the eosinophils not only involves the eye tissues but also it has been detected in peripheral blood.

In various forms of allergic eye disease, there is an increase of macrophages expressing CD68 and dendritic cells stained with CDla. Neutrophils are also recruited during the inflammation and remain in the tissues for a long time after the process.

Eye allergy mediators include tryptase, which is stored in mast cell granules and is increased in the tears of patients with vernal keratoconjunctivitis. In various forms of allergy, tumor necrosis factor alpha (TNF-α), histamine, quinines, and prostaglandin-D as well as products released by eosinophils can be found. The latter are not confined to eye tissues. (Bielory L. Am J Med. 2002 Dec 16;113 Suppl 9A:34S-7S).

Plasma levels of nerve growth factors (NGF) have been found in patients with vernal keratoconjunctivitis, which is also observed in people suffering from asthma (Lambiase et al., 1995 Invest Ophthalmol Vis Sci;36:2127-32). There are data that suggest a role of neurological and endocrinological factors in the pathogenesis and the clinical manifestation of allergic eye diseases.

Included among the mediators participating in the development of the allergic reaction are immunoglobulins. There are increased levels of total IgG in serum and tears in allergic conjunctivitis, particularly reflecting the presence of a systematic atopy. Allergen-specific IgE is frequently found in tears in seasonal allergic conjunctivitis and perennial allergic conjunctivitis.

IgA is the predominant immunoglobulin in mucosae, it is distributed in a different manner in bodily fluids. Secretory IgA is transported to the tear fluid through the epithelial cells derived from the secretory component, it is the first antibody detected in normal tears.

Patients with seasonal allergic conjunctivitis have low levels of pollen-specific IgG in tears. IgG also seems to play a role in the control of conjunctival infections.

In addition, adhesion molecules are fundamental for determining the degree and type of inflammatory cell infiltration in tissues. In a sensitized individual, the sole exposure of the conjunctiva to an allergen causes an overregulation of E-selectin after 30 minutes and of ICAM-1 and VCAM-1 after 4-24 hours in vascular endothelial cells. The increased expression of E-selectin in endothelial cells occurs in all types of allergic conjunctivitis and correlates with the degree of cell infiltration.

The allergic reaction occurs through a cascade of events in which the expression and regulation of these molecules are interrelated with one another. Mast cells are thus considered to be the main cell involved in the allergic reaction. In addition to the effect induced by the cross-linking of IgE-type antibodies with IgE receptors with a high affinity for the antigen, and by the release of several mediators, such as histamine (which causes the immediate degranulation of the mast cells), these cells also produce and release other factors such as tryptase (Leonardi A.Curr Opin Allergy Clin Immunol. 2005;5:464-472, Bonini S. Allergy. 2004;59:71-73). Similarly, eosinophils and their toxic proteins are increased and activated in the tears, conjunctiva and serum of all allergic eye diseases and are responsible for the corneal damages caused in these pathologies. Other inflammatory cells such as neutrophils also participate in the allergic response and contribute to the inflammation through the confinement of other cells and inflammatory mediators. Histamine is thus the most important and the majority mediator in the initial phase of an anaphylactic reaction, it can activate the T-cell suppressor, and therefore it can inhibit the production of IgE. This feedback process in which the effector molecule, in this case histamine, causes an inhibition of the molecule responsible for its release could explain how high levels of histamine in tears could be related to low levels of IgE and vice versa (McGill JI. et al., Br J Ophthalmol 1998; 82:1203-121).

In addition, the possibility that a nonspecific activation of the mast cells due to increased levels of their activating molecules such as tryptase has been described, and in a second phase once the mast cells are activated, the levels of tryptase would decrease and histamine would be released(Galatowicz G. et al. Clin Exp Allergy. 2007;37:1648-1656).

A diagnosis of eye allergy is established by means of medical records and a suitable physical examination carried out with a slit lamp by studying the conjunctiva, the cornea and the skin of the eyelids. As regards the laboratory tests, the concentration of IgE in tears and in serum can be determined: if it is greater in the former than in the latter, directed towards allergy. In conjunctival swabs, the presence of at least an eosinophil supports the diagnosis, but its absence does not exclude it since the prevalence of conjunctival eosinophils can vary between 20% and 80%.

The conjunctiva is the only tissue affected in up to 33% of the patients with eye allergy and both the serum tests (e.g. measurement of blood IgE) and skin tests (direct exposure of antigen with the skin) can be negative. Skin tests, such as the prick technique, consist of making a small puncture with a lancet through a drop of allergen deposited on the skin. Skin tests are not always positive in children and they do not always reflect real eye allergies. These false negatives in the diagnosis of eye allergy are due to the fact that the antigens are in contact with the conjunctiva of the eye and the allergic response is therefore more localized than systemic. Furthermore, it is complicated to perform them in children because they do not always collaborate and in addition may present skin hyperreactivity or eczema. For that reason, if all the previous examinations do not provide information in order to reach the diagnosis, the so-called conjunctival provocation tests could be performed.

Conjunctival provocation tests consist of applying drops of the suspected allergen in one of the conjunctivas and comparing it with the other for the possible onset of allergy symptoms, which usually occurs after 20 minutes. It is useful for confirming the conjunctival reactivity to allergens that have tested positive in skin tests and for those patients who have tested negative in skin tests and/or serum tests, or who have a clinical history of eye allergy, it further allows identifying the allergen causing the allergy. Nevertheless, it has several drawbacks. In the first place, at the time the test is performed the patients must be asymptomatic. Furthermore, the number of allergens that can be analyzed is limited. In addition, due to the high cost of allergens solubilized in water without preservatives, preparations of allergens which contain phenol and which can be irritating to the eye are used. These tests are not recommended in children because they may have severe systemic side effects, general itching, possibly causing bronchospasms and anaphylaxis.

The quality of life of the children with eye allergy and that of their family members is deeply affected, since allergic symptoms may affect the carrying out family activities, increase school absenteeism and interfere in sleep and in the ability to learn. Furthermore, in cases in which the subclinical eye allergy is confused with nervous tics, children may be treated with neuroleptics, which in these cases not only do not eliminate the tics, but they may also have negative side effects for the child. These include: reduced motivation, cognitive interference (memory and attention problems), interference with the school or work function, akinesia, akathisia, acute dystonia, tardive dyskinesia, anticholinergic effects (dry mouth, dilated pupils, blurred near vision, constipation, urine retention), and endocrinological effects.

Therefore, the development of new tests which allow the diagnosis of infant eye allergy that in a rapid, non-invasive and reliable manner without the possibility of causing side effects in children is necessary.

### Summary of the Invention

The inventors have found various markers which surprisingly allow diagnosing eye allergy in children. The assays conducted by the inventors have proven, as shown in Example 1, that the detection of the markers shown in Table 1 allows obtaining a hit rate of 94.44% when the level of all the markers is determined. Likewise, the determination of different combinations of 2 and 3 markers allows diagnosing infant eye allergy.

Therefore, in a first aspect the invention relates to an in vitro diagnostic method for diagnosing infant eye allergy in a child, which comprises:
(i) determining in a biological sample from said child the level of one or more markers selected from tryptase, histamine, ECF, MBP, ECP EDN IgE and E-selectin, and
(ii) comparing said level with the level of said marker or markers in a reference sample,
wherein an increase in the level of histamine, ECF, MBP, ECP, EDN and/or E-selectin determined in the sample from said child with respect to its level in said reference sample and/or a modulation in the level of IgE and/or tryptase with respect to its level in said reference sample is indicative of said child suffering from infant eye allergy.

In a second aspect, the invention relates to the use of a reagent capable of detecting and/or quantifying the level of a marker selected from the group consisting of tryptase, histamine, ECF, MBP, ECP, EDN, IgE and E-selectin for diagnosing infant eye allergy.

In another aspect, the invention relates to a product comprising a support and a reagent capable of detecting and/or quantifying the level of a marker selected from the group consisting of tryptase, histamine, ECF, MBP, ECP, EDN, IgE and E-selectin, wherein said reagent is fixed to said support

In another aspect, the invention relates to a kit comprising a reagent capable of detecting and/or quantifying the level of a marker selected from the group consisting of tryptase, histamine, ECF, MBP, ECP, EDN, IgE and E-selectin.

Likewise, the invention relates in other aspect to the use of said kit for the diagnosis of infant eye allergy.

### Brief Description of the Drawings

Figure 1 shows a graph corresponding to the comparative ROC curves of the markers ECF, ECP, EDN, IgE, histamine and tryptase.
Figure 2 shows a heat chart in which the hierarchical clustering of the samples and the markers is indicated. The numbers in the upper part identify each of the samples. The range of colors is indicative of the signal intensity and therefore of the amount of the marker. EA: eye allergy, CT: control.
Figure 3 shows the representation of the "support vector machine" multivariate analysis. The numbers in the upper part identify each of the samples. The range of colors is indicative of the signal intensity and therefore of the amount of the marker. EA: eye allergy, CT: control.
Figure 4 shows the representation of the discriminating multivariate analysis. Light dots: controls, dark dots: eye allergy.
Figure 5 shows the representation of PCA analysis. Light dots: controls, dark dots: eye allergy.

### Detailed Description of the Invention

The authors of the present invention have developed a diagnostic method for diagnosing infant eye allergy. Specifically, as observed in Example 1 of the present invention, the determination of the markers tryptase (Tps), histamine (His), ECF, MBP, ECP, EDN, IgE and E- selectin in tears allows the diagnosis of eye allergy in children.

Therefore in a first aspect, the invention relates to an in vitro diagnostic method for diagnosing infant eye allergy in a child, which comprises:
(i) determining in a biological sample from said child the level of one or more markers selected from tryptase, histamine, eotaxin (ECF), major basic protein (MBP), eosinophil cationic protein (ECP), eosinophil-derived neurotoxin protein (EDN), immunoglobulin E (IgE) and E-selectin, and
(ii) comparing said level with the level of said marker or markers in a reference sample,
wherein an increase in the level of histamine, ECF, MBP, ECP, EDN and E-selectin determined in the sample from said child with respect to its level in said reference sample and/or a modulation in the level of IgE and/or tryptase with respect to its level in said reference sample is indicative of said child suffering from infant eye allergy.

The diagnostic method of the invention is based on detecting the markers individually or on detecting combinations of said markers, for example, one, two, three, four, five, six, seven or even the eight markers previously mentioned.

The term "marker" will be used indistinctly throughout the invention together with the term "molecule/mediator" and refers to a protein or molecule.

In a particular embodiment, the levels of tryptase, histamine, ECF, MBP, ECP, EDN, IgE and E-selectin are determined.

Different combinations of two and three (or even more) markers provide good sensitivity, specificity and total hit values as shown in Table 4 for the diagnosis of infant eye allergy. In a particular embodiment, the levels of EDN, IgE and MBP are determined.

As it is used in the present invention, "infant eye allergy" or infant allergic conjunctivitis refers to an inflammation of the conjunctiva of a child caused by an allergenic agent. In the context of the present invention, the eye allergy maybe manifest or subclinical, i.e., it does not present symptoms or they are very minor. "Conjunctiva" refers to the transparent mucous membrane that covers the inner surface of the eyelid and the white part of the eyeball (the sclera). "Allergen" or allergenic agent refers to a substance, particle or organic body capable of causing an allergic reaction in a previously sensitized subject when he again comes into contact with said allergen.

"Child" refers to a human person between birth and puberty. Puberty is understood as the moment in which the physical and sexual characteristics mature due to hormonal changes. Therefore, in the present invention a child is considered to be a person of up to 14 years of age.

As it is used herein, the term "biological sample" refers to any biological sample which can be obtained from the eye of a patient, such as the conjunctiva, cornea, tears, aqueous humor, etc.

In a particular embodiment of the present invention, the biological sample is a tear. As it is used herein, "tear" refers to the aqueous liquid secreted by the tear glands to moisten and clean the conjunctiva of the eye by means of moving the eyelids. The tear sample can be obtained by conventional methods known by persons skilled in the art, for example, by means of an absorbent paper, a capillary, or a surgical spear. The sample can be obtained from previously diagnosed or undiagnosed subjects, or also from a subject undergoing treatment, or who has previously been treated for said pathology.

The initial step of the method of the invention for the in-vitro diagnosis of infant eye allergy in a biological sample from a child comprises determining the level of one or more markers selected from tryptase, histamine, ECF, MBP, ECP, EDN, IgE and E-selectin.

"Tryptase" refers to the majority protein contained in the mast cells used as a marker for activating said cells and therefore as an allergic response marker. This protein has several isoforms, alpha 1 (accession number P15157 SEQ ID NO:1), beta 1 (accession number Q15661, SEQ ID NO:2), beta2 (accession number P20231, SEQ ID NO:3 ), delta (accession number Q9BZJ3 SEQ ID NO:4) and gamma (accession number Q9NRR2, SEQ ID NO:5).

"Histamine" is understood as the local organic nitrogen compound, 4-(2-aminoethyl)-1,3-diazole, which participates in the immune response and is produced by the basophiles and mast cells which are found in the areas surrounding connective tissues.

"ECF", also referred to as eotaxin, refers to the protein with accession number P51671, SEQ ID NO:6, which acts as a marker of the allergic inflammatory reaction since it is an eosinophil chemotactic factor.

"MBP" refers to the largest basic protein of the eosinophil or proteoglycan of bone marrow specific eosinophil granules with accession number P13727, SEQ ID NO:7, the greatest constituent of the crystalloid heart of the specific eosinophil granules with toxic properties for cells.

"ECP" is understood as the eosinophil cationic protein with accession number P12724, SEQ ID NO: 8, a constituent of the matrix of the specific eosinophil granules with toxic properties for other types of cells.

"EDN" or EPX is understood as the eosinophil-derived neurotoxin protein with accession number P10153, SEQ ID NO: 9, characterized by having ribonuclease activity.

"IgE" refers to immunoglobulin E, an antibody involved in allergies, especially in type I hypersensitivity, such that the recognition of this antibody by the mast cells causes their degranulation.

"E-selectin" or CD62E refers to the protein with accession number P16581, SEQ ID NO: 10, which acts as an adhesion molecule that participates in the binding of the leucocytes to the activated vascular endothelium.

A person skilled in the art will recognize that the present invention relates not only to the specific markers mentioned in the present invention, but also to variants thereof. Said variants can be (i) a variant in which one or more amino acids is substituted by a conserved or non-conserved amino acid; (ii) a variant in which there is one or more modified amino acids, such as residues modified by the binding of substituent groups; (iii) variants in which the protein is a processing or splicing alternative and (iv) fragments in the case of a protein. The fragments include proteins generated through proteolytic cleavage. As is known in the state of the art, the "identity" between two proteins is determined by comparing the amino acid sequence of first and second proteins. Variants according to the present invention include amino acid sequences presenting at least 60%, 65%, 70%, 72%, 74%, 76%, 78%, 80%, 90%, or 95% similarity or identity with the original amino acid sequence. The degree of identity between two proteins is determined using computational algorithms and methods that are well known by persons skilled in the art. The identity between two amino acid sequences will preferably be determined using the BLASTP algorithm [BLASTManual, Altschul, S., et al., NCBI NLM NIH Bethesda, Md. 20894, Altschul, S., et al., J. Mol. Biol. 215: 403-410 (1990)]. The proteins of the invention may include post-translational modifications, such as glycosylation, acetylation, isoprenylation, myristoylation, proteolytic processing, etc.

In a particular embodiment of the present invention, the determination of the levels of the markers is carried out by means of an immunoassay.

Immunoassay is understood as different immunological techniques such as ELISA (Enzyme-Linked Immunosorbent Assay, Western-blot, RIA (radioimmunoassay), competitive EIA (competitive enzyme immunoassay), DAS-ELISA (Double Antibody Sandwich-ELISA), immunocytochemistry and immunohistochemistry techniques, techniques based on the use of biochips, of biomarkers, of biosensors or microarrays that include specific antibodies or assays based on the colloidal precipitation in formats such as dipsticks.

In another additional particular embodiment, the immunoassay is an ELISA. The ELISA technique is based on using antigens or antibodies labeled with enzymes, such that the conjugates formed between the target antigen and the labeled antibody results in the formation of enzymatically active complexes. Due to the fact that one of the components (the antigen or the labeled antibody) are immobilized on a support, the antibody-antigen complexes are immobilized on the support and can therefore be detected by adding a substrate which is converted by the enzyme into a product which is detectable, for example, by spectrophotometry or fluorometry.

A person skilled in the art will understand that the determination of the expression of the markers of the invention could be carried out by means of measuring the mRNA, a linear molecule containing information within the ribonucleotide sequence for directing protein synthesis, standard assays used for determining the expression levels of mRNA, such as RT-PCR, RNA protection analysis, Northern Blot process, *in-situ* hybridization, micromatrix technology and the like.

The final stage of the method of the invention for the *in vitro* diagnosis of infant eye allergy in a sample from a child, comprises comparing the amount of the described markers with the amount of said marker detected in a reference sample.

In the context of the present invention, the term "control or reference sample" is understood as the biological sample from a healthy subject or previous samples from the same individual, which is used for determining the variation of the expression levels of the markers used in the present invention. The variation of the expression levels in said control or reference sample can be determined before said levels are determined in the child that is to be diagnosed.

In the context of the present invention, it is said that the level of a marker is increased when the level of said marker is elevated with respect to the controls or reference values. According to the present invention, it is considered that the levels of a marker are greater than the levels of said markers in the reference sample when the levels of the marker in the sample from the subject are increased by at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

Likewise, in the context of the present invention it is said that the level of a marker is modulated when its level is greater than or less than the levels of said marker in the control or reference sample. In connection to this, it is said that the level of a marker is increased when the level of said marker is elevated with respect to the controls or reference values, as has previously been mentioned. Likewise, in the context of the present invention, it is said that the level of a marker is decreased or suppressed when the level of said marker is less than the controls or reference values. According to the present invention, the levels of a marker are considered to be less than the levels of said marker in the reference sample when the levels of the marker in the sample from the subject are decreased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

In a particular embodiment, the eye allergy is selected from seasonal eye allergy and perennial eye allergy. "Seasonal eye allergy" refers to eye allergies occurring with flare-ups related to the contact with the allergen. The term "perennial eye allergy" refers to eye allergies lasting for more than three weeks.

The method of the present invention further allows differentiating the patients that suffer eye allergy tics from those that suffer nervous tics; the patient can thus be administered the appropriate medical treatment. As it is used herein, the term "tic" refers to a temporary condition characterized by one or various brief, repetitive and difficult to control movements.

A particular embodiment of the invention relates to the use of a reagent capable of detecting and quantifying the level of a marker selected from the group consisting of tryptase, histamine, ECF, MBP, ECP, EDN, IgE and E-selectin for diagnosing infant eye allergy. Said "reagent" can be, but is not limited to, any type of antibody or immunoglobulin molecule or fragment thereof, such as polyclonal, monoclonal, human or humanized or recombinant antibodies, as well as single-chain antibodies, for example scFv constructs, or synthetic antibodies as such and belong to any of the following classes of immunoglobulin: IgG, IgM, IgE, IgA, and where deemed applicable, a subclass of any of the previously mentioned classes, for example the subclasses of the IgG class, such as IgGl, IgG2, IgG2a, IgG2b, IgG3 or IgGM. Furthermore, the reagents can also be antibody fragments such as Fv, Fab or F(ab')2 fragments or single-chain fragments such as scFv. Double-chain fragments such as Fv, Fab or F(ab')2 are preferred. The Fab and F(ab1)2 fragments do not have an Fc fragment contained in the intact antibodies. Such fragments can be produced from intact antibodies by means of proteolytic digestions using proteases such as papain (for producing Fab fragments) or pepsin (for producing F(ab')z fragments), or chemical oxidation.

In an additional embodiment, the reagent is fixed to a support. Physical supports include, but are not limited to, columns, filters, plastics, glass slides, silicone, microwells, nitrocellulose or PVDF membranes, and magnetic microspheres and others. A good support surface is chemically stable before and after coupling processes, allows good stain morphology, shows minimum non-specific binding, does not contribute to the background in detection systems and is compatible with different detection systems. The immobilization method used is reproducible, applicable to markers having different properties (size, hydrophilia, hydrophobia), maneuverable at high performance and automation and compatible with completely functional marker activity retention.

A particular embodiment of the present invention comprises a product comprising a support and a reagent capable of detecting and quantifying the level of a marker selected from the group consisting of tryptase, histamine, ECF, MBP, ECP, EDN, IgE and E-selectin, wherein said reagent is fixed to said support.

In a particular embodiment, the present invention relates to a kit comprising a reagent capable of detecting and quantifying the level of a marker selected from the group consisting of tryptase, histamine, ECF, MBP, ECP, EDN, IgE and E-selectin.

In another additional particular embodiment, the invention relates to a kit in which the reagent capable of detecting and quantifying the level of a marker selected from the group consisting of tryptase, histamine, ECF, MBP, ECP, EDN, IgE and E-selectin is fixed to a support.

In another particular embodiment, the invention relates to the use of said kit for the diagnosis of infant eye allergy.

In another additional particular embodiment, the invention relates to the use of said kit for differentiating eye allergy tics from nervous tics.

The invention is illustrated below based on the following examples provided as a non-limiting illustration of the scope of the invention.

### Example 1

### Identification of markers of infant eye allergy

### 1. Subjects

For the analysis of the levels of the markers in tears, children between 5 and 14 years of age with a clinical history of diagnosed allergy or symptoms of itching and tearing or sporadic redness and in whom the presence of seasonal allergic conjunctivitis (SAC) or perennial allergic conjunctivitis (PAC) was confirmed after examination with a slit lamp were included.

The causes for exclusion of the patients comprised the coexistence of another eye pathology, the coadministration of topical drugs, performing ophthalmologic surgical interventions 6 months prior or the existence of systemic pathologies affecting the eye surface.

The control group was formed by children 5 to 14 years of age without pathologies of the eye surface.

Twenty tear samples from children with allergy (17 with SAC and 3 with PAC) and 16 tear samples from healthy children (without a clinical history of allergy) used as controls were analyzed. The guardians of all the children involved in the study signed an informed consent giving their authorization for the collection of tear samples. Both the epidemiology and the biological data have been treated confidentially.

### 2. Collection of samples

The samples were collected by an ophthalmologist under a slit lamp strictly following the principles of the Declaration of Helsinki and after approval of the Research Ethics Committee of the Hospital de Cruces (Biscay).

Tear samples were collected from both eyes without prior anesthesia using polyvinyl acetate surgical spears (PVA ref 0525. OASIS, USA).

The surgical spear was placed on the edge of the lower eyelid without touching the eye surface to not cause irritation. The tip of the surgical spear was cut and processed to obtain the markers contained therein. Subsequently said tip was centrifuged at 15,000 rpm for 20 minutes. The tear obtained was frozen at -80°C until its subsequent analysis.

### 3. Quantification of the levels of the main markers involved in infant eye allergy

For the purpose of developing a diagnostic method for diagnosing infant eye allergy the markers considered to be involved in this pathology in tear samples from the subjects were analyzed. The markers which contribute to the diagnosis of infant eye allergy would be included in an initial diagnostic method prototype.

The levels of the markers were determined by means of sandwich ELISA (Enzyme-Linked ImmunoSorbent Assay). This technique consists of detecting an antigen, immobilized on a solid phase, by means of antibodies which indirectly cause a reaction the product of which can be spectrophotometrically measured. This principle has many of the properties of a good immunoassay: it is versatile, robust, and simple and easily separates the retained fraction from the free fraction by means of using the solid phase.

The quantification was performed in 96-well plastic microtiter plates treated in order to increase their molecule adsorption capacity and with optically clear well bottoms to allow for the optical density measurements in specific spectrophotometers referred to as ELISA readers. Unlike conventional spectrophotometers, ELISA readers, with the capacity to read all ultraviolet and visible wavelengths continuously, have filter systems which only allow reading one or a few wavelengths. These filters read at wavelengths necessary for determining the optical density of the most commonly used chromogens. All the markers in these assays were read at 450 nm.

The markers that were analyzed were: Histamine (His), tryptase (TPS), eosinophil chemotactic factor (ECF), major basic protein (MBP), eosinophil cationic protein (ECP), neurotoxin (EDN), IgE and E-selectin. Table 1 shows the values of the different markers obtained in the tears from children with SAC and PAC compared with control children.

**Table 1**

| Concentration values of the allergy mediators in tears from children with SAC and PAC compared with the values in tears from control children | | | |
|---|---|---|---|
| Allergy mediators | Concentration of allergy mediators in tears from children with SAC/PAC (ng/ml) | Concentration of allergy mediators in tears from controls (ng/ml) | p value<0.05 |
| TPS | 21.56 ± 13.79 | 32.9 ± 15.12 | 0.014 |
| MBP | 43.68 ± 37.2 | 24.92 ± 20.38 | 0.03 |
| HIS | 56 ± 130 | 4.4 ± 11 | 0.09 |
| ECP | 143.8 ± 209.9 | 5.5 ± 11.6 | 0.0041 |
| EDN | 322.5 ± 380.75 | 8.25 ± 12.25 | 0.0007 |
| E-Selectin | 8.95 ± 4.46 | 9.16 ± 6.60 | 0.456 |
| ECF | 9.28 ± 5.91 | 5.74 ± 7.45 | 0.06 |
| IgE | 8.97 ± 12.04 | 22.12 ± 19.9 | 0.014 |

| | | | |
|---|---|---|---|
| Significant value p<0.05. | | | |

### 4. Statistical Analysis

Once the concentration data for each of the markers is obtained by means of ELISA, two types of statistical analyses were performed. In the first place, each of the markers was analyzed by means of univariate statistics using ROC curves for the purpose of determining specificity and sensitivity individually. In the second place, multivariate hierarchical clustering analyses, a support vector machine and discriminant analysis were conducted to observe the robustness provided by the markers when considering them as a whole in the creation of predictive models. Likewise, the combination of markers which, utilized as a single panel, provide better prediction potential in diagnosis was also analyzed.

### 4.1 Univariate Statistical Analysis

A fundamental problem exists when treating the infant eye allergy data and it is mainly based on the fact that said pathology occurs through a series of intervals and, as a result, not all samples are taken in the same stages, nor should it be expected that children will go to the ophthalmologist's office during the allergy peak to be diagnosed.

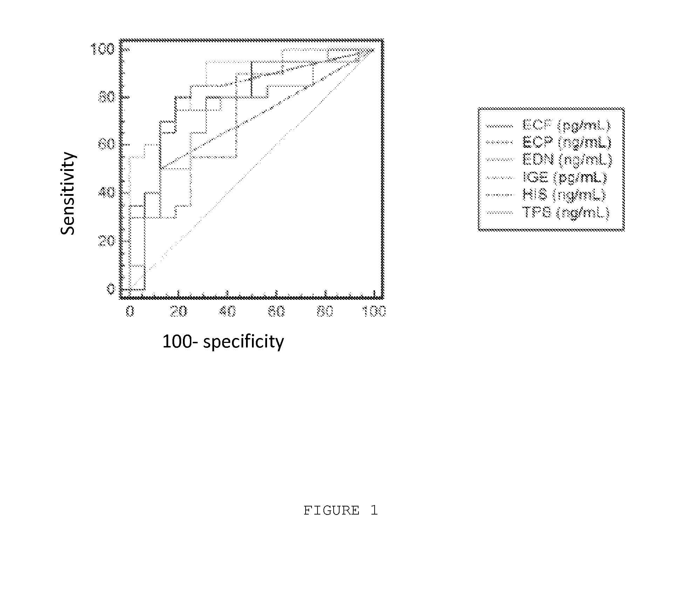

From the date of Table 1, the cutoff points or critical values indicating the separation value between the two groups (infant eye allergy and control) were determined in order to determine the sensitivity and specificity indicated in Table 2. The univariate statistical analyses performed included the Interactive Dot Diagram which allows obtaining the critical concentration value which provides maximum sensitivity and specificity (Table 2) and the comparative ROC curves (Figure 1).

**Table 2**

| Critical concentration values in tears (ng/ml) obtained for each marker | | | |
|---|---|---|---|
| Allergy mediators | Concentration in tears (ng/ml) | Sensitivity (%) | Specificity (%) |
| TPS | ≤23.8 | 75 | 68.7 |
| MBP | >12.55 | 85 | 50 |
| HIS | >5 | 50 | 87.5 |
| ECP | >3 | 80 | 81.2 |
| EDN | >3.5 | 95 | 68.7 |
| E-Selectin | >9.7 | 40 | 75 |
| ECF | >6.8 | 70 | 87.5 |
| IgE | ≤13.45 | 90 | 56.2 |

After the univariate statistical analyses it could be concluded that there was no marker which could act alone (according to the data collected from the ELISA assays) as a good and effective diagnostic tool for infant eye allergy. In most cases, when the markers are treated individually, there would be a risk (depending on the stage in which the patient goes to the doctor's office to be diagnosed) of failing 5% of the positive diagnoses (which is a very good value), but also of diagnosing 31.3% of the controls as positive. These values will be determined by the diagnosis with EDN, which is the best marker with the best ROC curve (Table 2).

For this reason it was essential to conduct a multivariate study in order to see the results more globally and to see the behavior of each class as a whole.

### 4.2 Multivariate Statistical Analysis

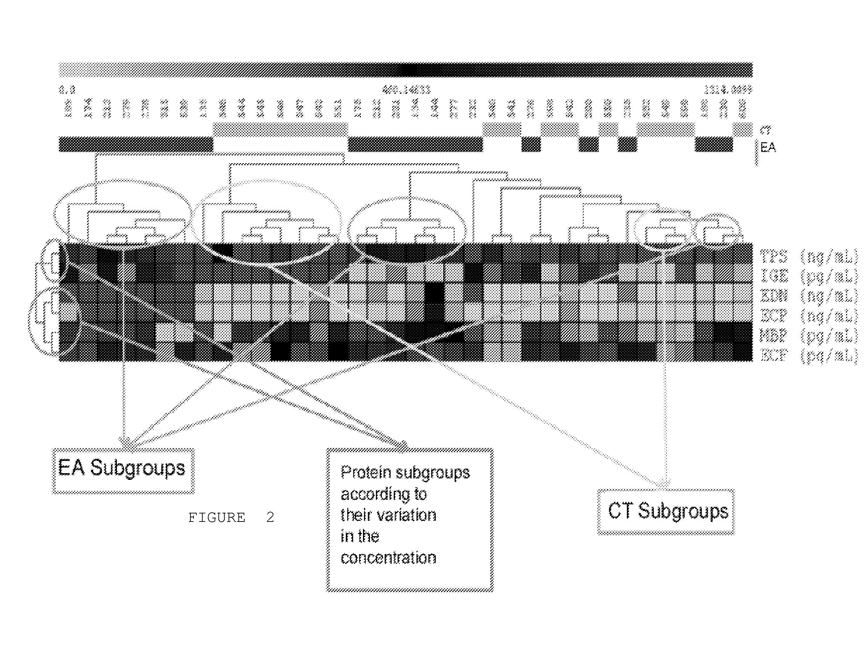

The data obtained were then treated with multivariate statistical analyses. In the first place, SAM-(Significance Analysis for Microarray) analysis was conducted, which determines from a number of variables, how many of them are significant and the number of false positives which may be introduced in the analysis. Six markers with significant differences with a 0% FDR (false discovery rate) were obtained. After eliminating the possible false positives, hierarchical clustering analysis of the most significant markers of the study was carried out. In this type of analysis the clustering between both the samples and between the markers (which of them have similar behaviors) can be seen and this is achieved as a result of a hierarchical clustering statistical analysis based on the measurement of the similarity distance between both the markers and between the samples according to Pearson's correlation. As observed in Figure 2 there was not a perfect clustering between the two classes of infant eye allergy (EA) and control (CT), although there were several different types of behaviors among the samples, and also an isolated case (samples 276, 280, 235, and, in addition, samples 188 and 230 for the case of EA).

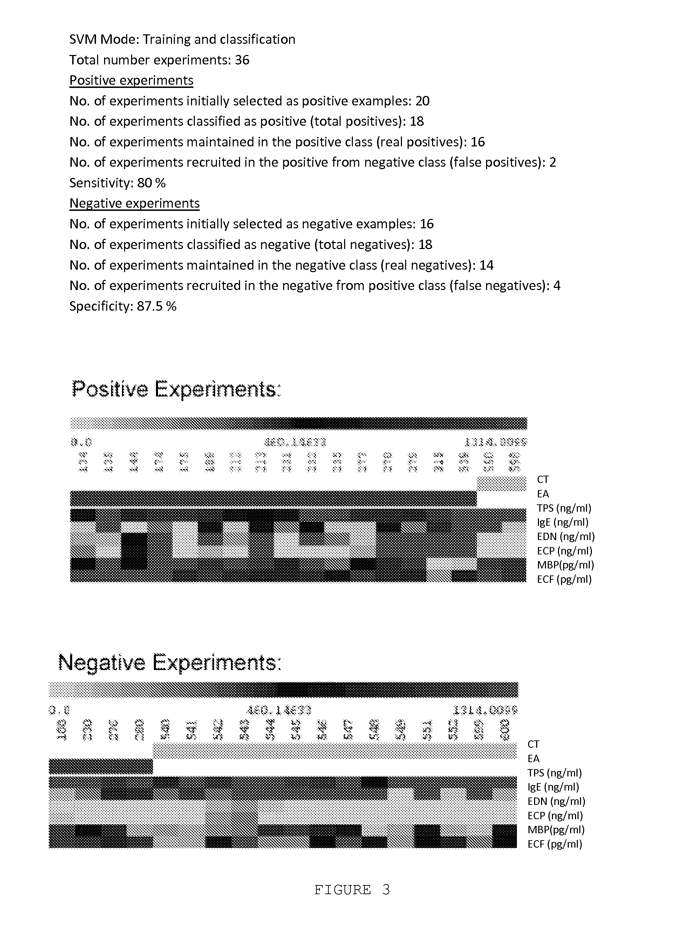

The SVM (support vector machine) is utilized for classifying samples after conducting training with the data by way of learning. By learning from different groups of data, a classification model is generated which allows deciding to which group a new unknown sample belongs. To carry out this analysis only the 6 most statistically significant markers without including false positives were used, specifically: TPS, IgE, EDN, ECP, MBP and ECF. As a result of this statistical technique, it was proved that it is possible to obtain 80% sensitivity and 87.5% specificity (Figure 3). Samples 550 and 598 appeared, after this analysis, as false positives, meanwhile samples 188, 230, 276 and 280, as false negatives.

Once the multivariate analyses were conducted it was found that there was no established group behavior for allergic individuals, but rather different clusters appeared which certainly correspond to different stages of the pathology.

### 4.3 Discriminant Statistical Analysis

The data were then analyzed by means of discriminant analysis. In this type of analysis the samples were classified by means of an unsupervised method. This means that at the beginning of the analysis the group to which each sample belonged was indicated, but for the classification thereof, its behavior was compared with each of the different classes that were included among the data (in this case two) without taking itself into account. Once the classification result was obtained for each sample, it was validated with respect to that initially indicated (clinically obtained).

This type of analysis, in addition to giving a statistical group classification model as a result, reduces the data to PCA (Principal Component Analysis) style, but revealing those more discriminating factors between the groups, obtaining a graphical representation that shows the distance between the samples.

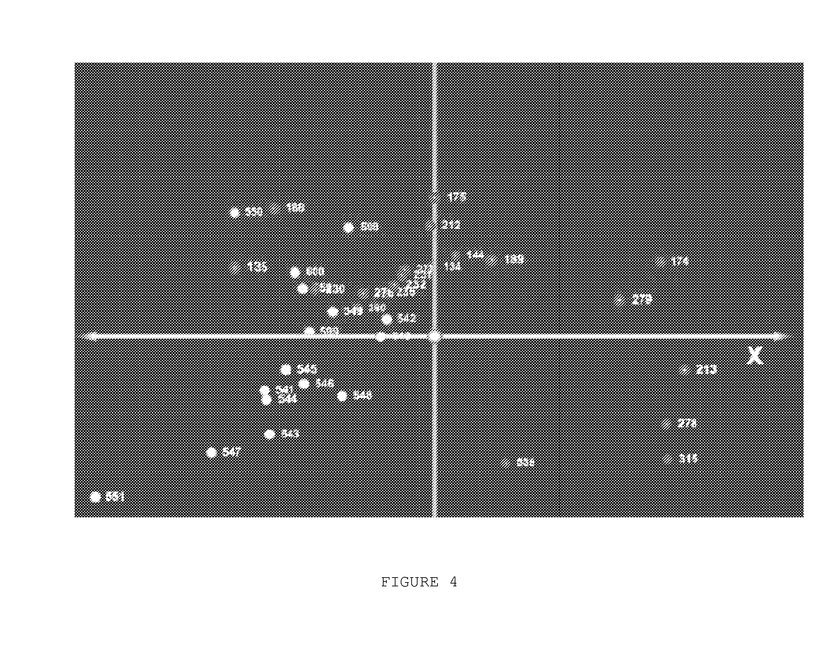

These results, shown in Figure 4, do not differ much from the results obtained by means of hierarchical clustering shown in Figure 2 because there is no complete clustering of patients with eye allergy, but rather they are in small clusters.

PCA analysis is a statistical analysis based on extracting the most representative values of each group and the reduction in number thereof by means of numerical transformations without information loss. Unlike discriminant analysis, PCA is an exploratory data analysis but it does not generate a predictive model.

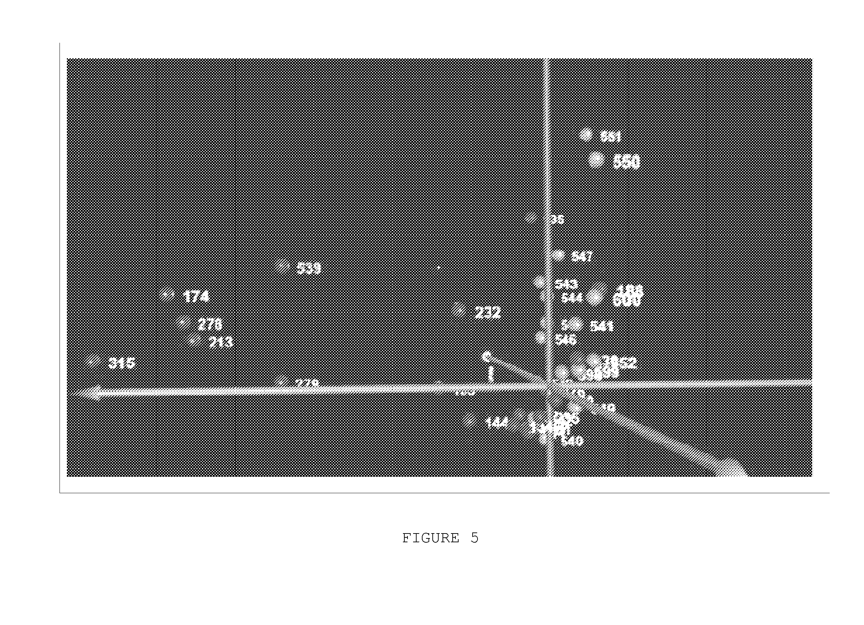

Although the separation between the samples is less than what is obtained in the case of discriminant analysis (Figure 5), the results allow the same conclusion, the existence of several different behaviors in the infant eye allergy group.

On the other hand, the logistic regression analyses, which in turn act as a predictive model, indicated, in the first place, that the statistical significance of the markers was irrelevant given that, since there was a different behavior of the same group for each marker, it made the deviations large and the variances between the groups small. This was seen especially when introducing variables without statistical significance into the analysis (Table 2), and observing that the diagnostic hit rate increased. On the other hand, logistic regression also determined that by using all the markers for the diagnosis, 94.4% of the cases, both healthy as well as allergy (Table 3), could be correctly predicted.

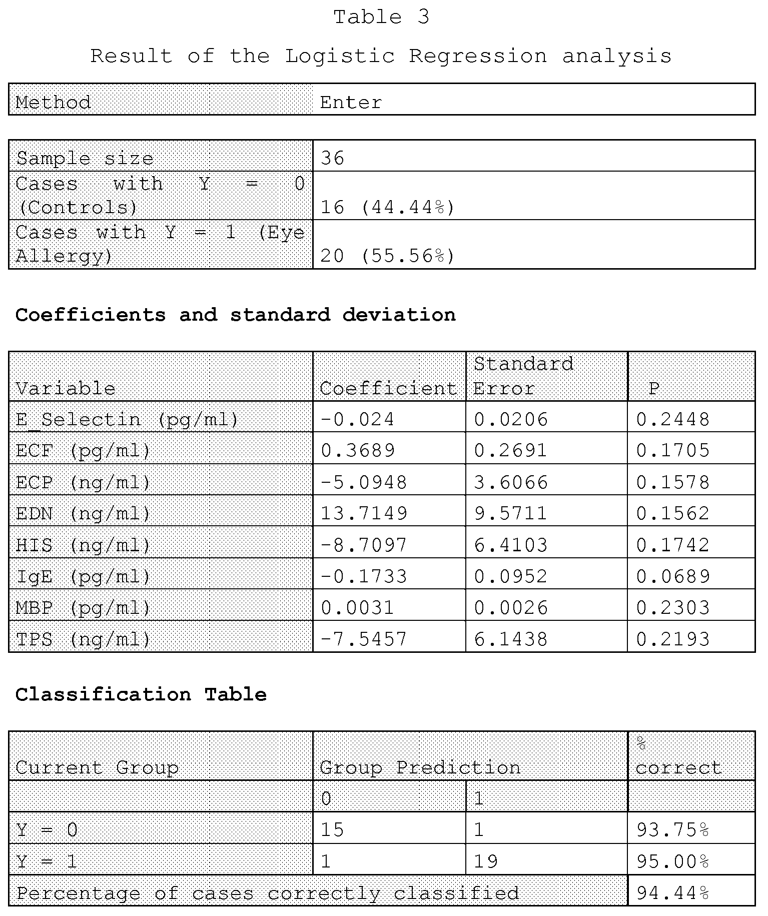

In the same manner, as is shown in Table 4, the use of the combination of different markers allowed obtaining a sensitivity of 80% to 93.33%, a specificity of 70% to 90% and a total hit rate from 74.14% to 88.57%. The determination of the levels of EDN, IgE and MBP allowed obtaining a sensitivity of 86.67%, a specificity of 90% and a total hit rate of 88.57%, indicating the best specificity and an elevated sensitivity, in addition to providing the highest number of total hits.

**Table 4**

| **ANALYSIS OF THE COMBINATION OF BIOMARKERS FOR DIAGNOSING INFANT EYE ALLERGY** | | | |
|---|---|---|---|
| **Combination of markers** | **Sensitivity (%)** | **Specificity (%)** | **% total hits** |
| EDN + E-Selectin | 86.67 | 70 | 77.14 |
| EDN + ECF | 80 | 70 | 74.29 |
| EDN + ECP | 86.67 | 70 | 77.14 |
| EDN + His | 86.67 | 70 | 77.14 |
| EDN + IgE | 86.67 | 80 | 82.86 |
| EDN + MBP | 86.67 | 75 | 80 |
| EDN + TPS | 80 | 75 | 74.14 |
| EDN + E-selectin + ECF | 80 | 70 | 74.29 |
| EDN + E-selectin + ECP | 86.67 | 70 | 77.14 |
| EDN + E-selectin + HIS | 86.67 | 70 | 77.14 |
| EDN + E-selectin + IgE | 93.33 | 80 | 85.71 |
| EDN + E-selectin + MBP | 80 | 80 | 80 |
| EDN + E-selectin + TPS | 86.67 | 75 | 80 |
| EDN + ECF + ECP | 80 | 70 | 74.29 |
| EDN + ECF + His | 80 | 70 | 74.29 |
| EDN + ECF + IgE | 86.67 | 80 | 82.86 |
| EDN + ECF + MBP | 80 | 75 | 77.14 |
| EDN + ECF + TPS | 80 | 80 | 80 |
| EDN + ECP + His | 86.67 | 70 | 77.14 |
| EDN + ECP + IgE | 93.33 | 80 | 85.71 |
| EDN + ECP + MBP | 86.67 | 75 | 80 |
| EDN + ECP + TPS | 86.67 | 70 | 77.14 |
| EDN + His + IgE | 86.67 | 80 | 82.86 |
| EDN + His + MBP | 80 | 80 | 80 |
| EDN + His + TPS | 86.67 | 70 | 77.14 |
| EDN + IgE + MBP | 86.67 | 90 | 88.57 |
| EDN + IgE + TPS | 86.67 | 75 | 80 |
| EDN + MBP + TPS | 80 | 80 | 80 |

## Claims

1. An *in vitro* diagnostic method for diagnosing infant eye allergy in a child which comprises:
(iii) determining in a biological sample from said child the level of one or more markers selected from tryptase, histamine, eotaxin (ECF), major basic protein (MBP), eosinophil cationic protein (ECP), eosinophil-derived neurotoxin protein (EDN), immunoglobulin E (IgE) and E-selectin.
(iv) comparing said level with the level of said marker or markers in a reference sample,
wherein an increase in the level of histamine, ECF, MBP, ECP, EDN and/or E-selectin determined in the sample from said child with respect to its level in said reference sample and/or a modulation in the level of IgE and/or tryptase with respect to its level in said reference sample is indicative of said child suffering from infant eye allergy.

2. The method according to claim 1, wherein the levels of tryptase, histamine, ECF, MBP, ECP, EDN, IgE and E-selectin are determined.

3. The method according to claim 1, wherein the levels of EDN, IgE and MBP are determined.

4. The method according to any of the claims 1 to 3, wherein said biological sample is a tear.

5. The method according to any of claims 1 to 4, wherein the levels of the markers are determined by the means of an immunoassay.

6. The method according to claim 5, wherein said immunoassay is an ELISA.

7. The method according to any of the previous claims, wherein said eye allergy is selected from seasonal eye allergy and perennial eye allergy

8. Use of a reagent capable of detecting and/or quantifying the level of a marker selected from the group consisting of tryptase, histamine, ECF, MBP, ECP, EDN, IgE and E-selectin for diagnosing infant eye allergy.

9. The use according to claim 8, wherein said reagent capable of detecting and/or quantifying the level of a marker selected from the group consisting of tryptase, histamine, ECF, MBP, ECP, EDN, IgE and E-selectin is fixed to a support.

10. A product comprising a support and a reagent capable of detecting and/or quantifying the level of a marker selected from the group consisting of tryptase, histamine, ECF, MBP, ECP, EDN, IgE and E-selectin, wherein said reagent is fixed to said support.

11. A kit comprising a reagent capable of detecting and/or quantifying the level of a marker selected from the group consisting of tryptase, histamine, ECF, MBP, ECP, EDN, IgE and E-selectin, and combinations thereof.

12. The kit according to claim 11, wherein said reagent capable of detecting and/or quantifying the level of a marker selected from the group consisting of tryptase, histamine, ECF, MBP, ECP, EDN, IgE and E-selectin is fixed to a support.

13. Use of a kit according to claim 11 or 12, for the diagnosis of infant eye allergy.

14. Use of a kit according to claim 11 or 12, for the differentiation of eye allergy tics from nervous tics.


SEQUENCE LISTING

<110>  BIOFTALMIK S.L.

<120>  MÉTODO DE DIAGNÓSTICO DE  ALERGIA OCULAR INFANTIL

<130>  P6388EP00

<160>  10

<170>  PatentIn version 3.5

<210>  1
<211>  275
<212>  PRT
<213>  Homo sapiens

<400>  1

Met Leu Ser Leu Leu Leu Leu Ala Leu Pro Val Leu Ala Ser Arg Ala
1               5                   10                  15

Tyr Ala Ala Pro Ala Pro Val Gln Ala Leu Gln Gln Ala Gly Ile Val
            20                  25                  30

Gly Gly Gln Glu Ala Pro Arg Ser Lys Trp Pro Trp Gln Val Ser Leu
        35                  40                  45

Arg Val Arg Asp Arg Tyr Trp Met His Phe Cys Gly Gly Ser Leu Ile
    50                  55                  60

His Pro Gln Trp Val Leu Thr Ala Ala His Cys Leu Gly Pro Asp Val
65                  70                  75                  80

Lys Asp Leu Ala Thr Leu Arg Val Gln Leu Arg Glu Gln His Leu Tyr
                85                  90                  95

Tyr Gln Asp Gln Leu Leu Pro Val Ser Arg Ile Ile Val His Pro Gln
            100                 105                 110

Phe Tyr Ile Ile Gln Thr Gly Ala Asp Ile Ala Leu Leu Glu Leu Glu
        115                 120                 125

Glu Pro Val Asn Ile Ser Ser Arg Val His Thr Val Met Leu Pro Pro
    130                 135                 140

Ala Ser Glu Thr Phe Pro Pro Gly Met Pro Cys Trp Val Thr Gly Trp
145                 150                 155                 160

Gly Asp Val Asp Asn Asp Glu Pro Leu Pro Pro Pro Phe Pro Leu Lys
                165                 170                 175

Gln Val Lys Val Pro Ile Met Glu Asn His Ile Cys Asp Ala Lys Tyr
            180                 185                 190

His Leu Gly Ala Tyr Thr Gly Asp Asp Val Arg Ile Ile Arg Asp Asp
        195                 200                 205

Met Leu Cys Ala Gly Asn Thr Arg Arg Asp Ser Cys Lys Gly Asp Ser
    210                 215                 220

Gly Gly Pro Leu Val Cys Lys Val Asn Gly Thr Trp Leu Gln Ala Gly
225                 230                 235                 240

Val Val Ser Trp Asp Glu Gly Cys Ala Gln Pro Asn Arg Pro Gly Ile
                245                 250                 255

Tyr Thr Arg Val Thr Tyr Tyr Leu Asp Trp Ile His His Tyr Val Pro
            260                 265                 270

Lys Lys Pro
        275

<210>  2
<211>  266
<212>  PRT
<213>  Homo sapiens

<400>  2

Met Leu Asn Leu Leu Leu Leu Ala Leu Pro Val Leu Ala Ser Arg Ala
1               5                   10                  15

Tyr Ala Ala Pro Ala Pro Gly Gln Ala Leu Gln Arg Val Gly Ile Val
            20                  25                  30

Gly Gly Gln Glu Ala Pro Arg Ser Lys Trp Pro Trp Gln Val Ser Leu
        35                  40                  45

Arg Val His Gly Pro Tyr Trp Met His Phe Cys Gly Gly Ser Leu Ile
    50                  55                  60

His Pro Gln Trp Val Leu Thr Ala Ala His Cys Val Gly Pro Val Gln
65                  70                  75                  80

Leu Arg Glu Gln His Leu Tyr Tyr Gln Asp Gln Leu Leu Pro Val Ser
                85                  90                  95

Arg Ile Ile Val His Pro Gln Phe Tyr Thr Ala Gln Ile Gly Ala Asp
            100                 105                 110

Ile Ala Leu Leu Glu Leu Glu Glu Pro Val Asn Val Ser Ser His Val
        115                 120                 125

His Thr Val Thr Leu Pro Pro Ala Ser Glu Thr Phe Pro Pro Gly Met
    130                 135                 140

Pro Cys Trp Val Thr Gly Trp Gly Asp Val Asp Asn Asp Glu Arg Leu
145                 150                 155                 160

Pro Pro Pro Phe Pro Leu Lys Gln Val Lys Val Pro Ile Met Glu Asn
                165                 170                 175

His Ile Cys Asp Ala Lys Tyr His Leu Gly Ala Tyr Thr Gly Asp Asp
            180                 185                 190

Val Arg Ile Val Arg Asp Asp Met Leu Cys Ala Gly Asn Thr Arg Arg
        195                 200                 205

Asp Ser Cys Gln Gly Asp Ser Gly Gly Pro Leu Val Cys Lys Val Asn
    210                 215                 220

Gly Thr Trp Leu Gln Ala Gly Val Val Ser Trp Gly Glu Gly Cys Ala
225                 230                 235                 240

Gln Pro Asn Arg Pro Gly Ile Tyr Thr Arg Val Thr Tyr Tyr Leu Asp
                245                 250                 255

Trp Ile His His Tyr Val Pro Lys Lys Pro
            260                 265

<210>  3
<211>  275
<212>  PRT
<213>  Homo sapiens

<400>  3

Met Leu Asn Leu Leu Leu Leu Ala Leu Pro Val Leu Ala Ser Arg Ala
1               5                   10                  15

Tyr Ala Ala Pro Ala Pro Gly Gln Ala Leu Gln Arg Val Gly Ile Val
            20                  25                  30

Gly Gly Gln Glu Ala Pro Arg Ser Lys Trp Pro Trp Gln Val Ser Leu
        35                  40                  45

Arg Val His Gly Pro Tyr Trp Met His Phe Cys Gly Gly Ser Leu Ile
    50                  55                  60

His Pro Gln Trp Val Leu Thr Ala Ala His Cys Val Gly Pro Asp Val
65                  70                  75                  80

Lys Asp Leu Ala Ala Leu Arg Val Gln Leu Arg Glu Gln His Leu Tyr
                85                  90                  95

Tyr Gln Asp Gln Leu Leu Pro Val Ser Arg Ile Ile Val His Pro Gln
            100                 105                 110

Phe Tyr Thr Ala Gln Ile Gly Ala Asp Ile Ala Leu Leu Glu Leu Glu
        115                 120                 125

Glu Pro Val Asn Val Ser Ser His Val His Thr Val Thr Leu Pro Pro
    130                 135                 140

Ala Ser Glu Thr Phe Pro Pro Gly Met Pro Cys Trp Val Thr Gly Trp
145                 150                 155                 160

Gly Asp Val Asp Asn Asp Glu Arg Leu Pro Pro Pro Phe Pro Leu Lys
                165                 170                 175

Gln Val Lys Val Pro Ile Met Glu Asn His Ile Cys Asp Ala Lys Tyr
            180                 185                 190

His Leu Gly Ala Tyr Thr Gly Asp Asp Val Arg Ile Val Arg Asp Asp
        195                 200                 205

Met Leu Cys Ala Gly Asn Thr Arg Arg Asp Ser Cys Gln Gly Asp Ser
    210                 215                 220

Gly Gly Pro Leu Val Cys Lys Val Asn Gly Thr Trp Leu Gln Ala Gly
225                 230                 235                 240

Val Val Ser Trp Gly Glu Gly Cys Ala Gln Pro Asn Arg Pro Gly Ile
                245                 250                 255

Tyr Thr Arg Val Thr Tyr Tyr Leu Asp Trp Ile His His Tyr Val Pro
            260                 265                 270

Lys Lys Pro
        275

<210>  4
<211>  242
<212>  PRT
<213>  Homo sapiens

<400>  4

Met Leu Leu Leu Ala Pro Gln Met Leu Ser Leu Leu Leu Leu Ala Leu
1               5                   10                  15

Pro Val Leu Ala Ser Pro Ala Tyr Val Ala Pro Ala Pro Gly Gln Ala
            20                  25                  30

Leu Gln Gln Thr Gly Ile Val Gly Gly Gln Glu Ala Pro Arg Ser Lys
        35                  40                  45

Trp Pro Trp Gln Val Ser Leu Arg Val Arg Gly Pro Tyr Trp Met His
    50                  55                  60

Phe Cys Gly Gly Ser Leu Ile His Pro Gln Trp Val Leu Thr Ala Ala
65                  70                  75                  80

His Cys Val Glu Pro Asp Ile Lys Asp Leu Ala Ala Leu Arg Val Gln
                85                  90                  95

Leu Arg Glu Gln His Leu Tyr Tyr Gln Asp Gln Leu Leu Pro Val Ser
            100                 105                 110

Arg Ile Ile Val His Pro Gln Phe Tyr Ile Ile Gln Thr Gly Ala Asp
        115                 120                 125

Ile Ala Leu Leu Glu Leu Glu Glu Pro Val Asn Ile Ser Ser His Ile
    130                 135                 140

His Thr Val Thr Leu Pro Pro Ala Ser Glu Thr Phe Pro Pro Gly Met
145                 150                 155                 160

Pro Cys Trp Val Thr Gly Trp Gly Asp Val Asp Asn Asn Val His Leu
                165                 170                 175

Pro Pro Pro Tyr Pro Leu Lys Glu Val Glu Val Pro Val Val Glu Asn
            180                 185                 190

His Leu Cys Asn Ala Glu Tyr His Thr Gly Leu His Thr Gly His Ser
        195                 200                 205

Phe Gln Ile Val Arg Asp Asp Met Leu Cys Ala Gly Ser Glu Asn His
    210                 215                 220

Asp Ser Cys Gln Gly Asp Ser Gly Gly Pro Leu Val Cys Lys Val Asn
225                 230                 235                 240

Gly Thr

<210>  5
<211>  321
<212>  PRT
<213>  Homo sapiens

<400>  5

Met Ala Leu Gly Ala Cys Gly Leu Leu Leu Leu Leu Ala Val Pro Gly
1               5                   10                  15

Val Ser Leu Arg Thr Leu Gln Pro Gly Cys Gly Arg Pro Gln Val Ser
            20                  25                  30

Asp Ala Gly Gly Arg Ile Val Gly Gly His Ala Ala Pro Ala Gly Ala
        35                  40                  45

Trp Pro Trp Gln Ala Ser Leu Arg Leu Arg Arg Met His Val Cys Gly
    50                  55                  60

Gly Ser Leu Leu Ser Pro Gln Trp Val Leu Thr Ala Ala His Cys Phe
65                  70                  75                  80

Ser Gly Ser Leu Asn Ser Ser Asp Tyr Gln Val His Leu Gly Glu Leu
                85                  90                  95

Glu Ile Thr Leu Ser Pro His Phe Ser Thr Val Arg Gln Ile Ile Leu
            100                 105                 110

His Ser Ser Pro Ser Gly Gln Pro Gly Thr Ser Gly Asp Ile Ala Leu
        115                 120                 125

Val Glu Leu Ser Val Pro Val Thr Leu Ser Ser Arg Ile Leu Pro Val
    130                 135                 140

Cys Leu Pro Glu Ala Ser Asp Asp Phe Cys Pro Gly Ile Arg Cys Trp
145                 150                 155                 160

Val Thr Gly Trp Gly Tyr Thr Arg Glu Gly Glu Pro Leu Pro Pro Pro
                165                 170                 175

Tyr Ser Leu Arg Glu Val Lys Val Ser Val Val Asp Thr Glu Thr Cys
            180                 185                 190

Arg Arg Asp Tyr Pro Gly Pro Gly Gly Ser Ile Leu Gln Pro Asp Met
        195                 200                 205

Leu Cys Ala Arg Gly Pro Gly Asp Ala Cys Gln Asp Asp Ser Gly Gly
    210                 215                 220

Pro Leu Val Cys Gln Val Asn Gly Ala Trp Val Gln Ala Gly Thr Val
225                 230                 235                 240

Ser Trp Gly Glu Gly Cys Gly Arg Pro Asn Arg Pro Gly Val Tyr Thr
                245                 250                 255

Arg Val Pro Ala Tyr Val Asn Trp Ile Arg Arg His Ile Thr Ala Ser
            260                 265                 270

Gly Gly Ser Glu Ser Gly Tyr Pro Arg Leu Pro Leu Leu Ala Gly Leu
        275                 280                 285

Phe Leu Pro Gly Leu Phe Leu Leu Leu Val Ser Cys Val Leu Leu Ala
    290                 295                 300

Lys Cys Leu Leu His Pro Ser Ala Asp Gly Thr Pro Phe Pro Ala Pro
305                 310                 315                 320

Asp

<210>  6
<211>  97
<212>  PRT
<213>  Homo sapiens

<400>  6

Met Lys Val Ser Ala Ala Leu Leu Trp Leu Leu Leu Ile Ala Ala Ala
1               5                   10                  15

Phe Ser Pro Gln Gly Leu Ala Gly Pro Ala Ser Val Pro Thr Thr Cys
            20                  25                  30

Cys Phe Asn Leu Ala Asn Arg Lys Ile Pro Leu Gln Arg Leu Glu Ser
        35                  40                  45

Tyr Arg Arg Ile Thr Ser Gly Lys Cys Pro Gln Lys Ala Val Ile Phe
    50                  55                  60

Lys Thr Lys Leu Ala Lys Asp Ile Cys Ala Asp Pro Lys Lys Lys Trp
65                  70                  75                  80

Val Gln Asp Ser Met Lys Tyr Leu Asp Gln Lys Ser Pro Thr Pro Lys
                85                  90                  95

Pro

<210>  7
<211>  222
<212>  PRT
<213>  Homo sapiens

<400>  7

Met Lys Leu Pro Leu Leu Leu Ala Leu Leu Phe Gly Ala Val Ser Ala
1               5                   10                  15

Leu His Leu Arg Ser Glu Thr Ser Thr Phe Glu Thr Pro Leu Gly Ala
            20                  25                  30

Lys Thr Leu Pro Glu Asp Glu Glu Thr Pro Glu Gln Glu Met Glu Glu
        35                  40                  45

Thr Pro Cys Arg Glu Leu Glu Glu Glu Glu Glu Trp Gly Ser Gly Ser
    50                  55                  60

Glu Asp Ala Ser Lys Lys Asp Gly Ala Val Glu Ser Ile Ser Val Pro
65                  70                  75                  80

Asp Met Val Asp Lys Asn Leu Thr Cys Pro Glu Glu Glu Asp Thr Val
                85                  90                  95

Lys Val Val Gly Ile Pro Gly Cys Gln Thr Cys Arg Tyr Leu Leu Val
            100                 105                 110

Arg Ser Leu Gln Thr Phe Ser Gln Ala Trp Phe Thr Cys Arg Arg Cys
        115                 120                 125

Tyr Arg Gly Asn Leu Val Ser Ile His Asn Phe Asn Ile Asn Tyr Arg
    130                 135                 140

Ile Gln Cys Ser Val Ser Ala Leu Asn Gln Gly Gln Val Trp Ile Gly
145                 150                 155                 160

Gly Arg Ile Thr Gly Ser Gly Arg Cys Arg Arg Phe Gln Trp Val Asp
                165                 170                 175

Gly Ser Arg Trp Asn Phe Ala Tyr Trp Ala Ala His Gln Pro Trp Ser
            180                 185                 190

Arg Gly Gly His Cys Val Ala Leu Cys Thr Arg Gly Gly His Trp Arg
        195                 200                 205

Arg Ala His Cys Leu Arg Arg Leu Pro Phe Ile Cys Ser Tyr
    210                 215                 220

<210>  8
<211>  160
<212>  PRT
<213>  Homo sapiens

<400>  8

Met Val Pro Lys Leu Phe Thr Ser Gln Ile Cys Leu Leu Leu Leu Leu
1               5                   10                  15

Gly Leu Met Gly Val Glu Gly Ser Leu His Ala Arg Pro Pro Gln Phe
            20                  25                  30

Thr Arg Ala Gln Trp Phe Ala Ile Gln His Ile Ser Leu Asn Pro Pro
        35                  40                  45

Arg Cys Thr Ile Ala Met Arg Ala Ile Asn Asn Tyr Arg Trp Arg Cys
    50                  55                  60

Lys Asn Gln Asn Thr Phe Leu Arg Thr Thr Phe Ala Asn Val Val Asn
65                  70                  75                  80

Val Cys Gly Asn Gln Ser Ile Arg Cys Pro His Asn Arg Thr Leu Asn
                85                  90                  95

Asn Cys His Arg Ser Arg Phe Arg Val Pro Leu Leu His Cys Asp Leu
            100                 105                 110

Ile Asn Pro Gly Ala Gln Asn Ile Ser Asn Cys Thr Tyr Ala Asp Arg
        115                 120                 125

Pro Gly Arg Arg Phe Tyr Val Val Ala Cys Asp Asn Arg Asp Pro Arg
    130                 135                 140

Asp Ser Pro Arg Tyr Pro Val Val Pro Val His Leu Asp Thr Thr Ile
145                 150                 155                 160

<210>  9
<211>  161
<212>  PRT
<213>  Homo sapiens

<400>  9

Met Val Pro Lys Leu Phe Thr Ser Gln Ile Cys Leu Leu Leu Leu Leu
1               5                   10                  15

Gly Leu Leu Ala Val Glu Gly Ser Leu His Val Lys Pro Pro Gln Phe
            20                  25                  30

Thr Trp Ala Gln Trp Phe Glu Thr Gln His Ile Asn Met Thr Ser Gln
        35                  40                  45

Gln Cys Thr Asn Ala Met Gln Val Ile Asn Asn Tyr Gln Arg Arg Cys
    50                  55                  60

Lys Asn Gln Asn Thr Phe Leu Leu Thr Thr Phe Ala Asn Val Val Asn
65                  70                  75                  80

Val Cys Gly Asn Pro Asn Met Thr Cys Pro Ser Asn Lys Thr Arg Lys
                85                  90                  95

Asn Cys His His Ser Gly Ser Gln Val Pro Leu Ile His Cys Asn Leu
            100                 105                 110

Thr Thr Pro Ser Pro Gln Asn Ile Ser Asn Cys Arg Tyr Ala Gln Thr
        115                 120                 125

Pro Ala Asn Met Phe Tyr Ile Val Ala Cys Asp Asn Arg Asp Gln Arg
    130                 135                 140

Arg Asp Pro Pro Gln Tyr Pro Val Val Pro Val His Leu Asp Arg Ile
145                 150                 155                 160

Ile

<210>  10
<211>  610
<212>  PRT
<213>  Homo sapiens

<400>  10

Met Ile Ala Ser Gln Phe Leu Ser Ala Leu Thr Leu Val Leu Leu Ile
1               5                   10                  15

Lys Glu Ser Gly Ala Trp Ser Tyr Asn Thr Ser Thr Glu Ala Met Thr
            20                  25                  30

Tyr Asp Glu Ala Ser Ala Tyr Cys Gln Gln Arg Tyr Thr His Leu Val
        35                  40                  45

Ala Ile Gln Asn Lys Glu Glu Ile Glu Tyr Leu Asn Ser Ile Leu Ser
    50                  55                  60

Tyr Ser Pro Ser Tyr Tyr Trp Ile Gly Ile Arg Lys Val Asn Asn Val
65                  70                  75                  80

Trp Val Trp Val Gly Thr Gln Lys Pro Leu Thr Glu Glu Ala Lys Asn
                85                  90                  95

Trp Ala Pro Gly Glu Pro Asn Asn Arg Gln Lys Asp Glu Asp Cys Val
            100                 105                 110

Glu Ile Tyr Ile Lys Arg Glu Lys Asp Val Gly Met Trp Asn Asp Glu
        115                 120                 125

Arg Cys Ser Lys Lys Lys Leu Ala Leu Cys Tyr Thr Ala Ala Cys Thr
    130                 135                 140

Asn Thr Ser Cys Ser Gly His Gly Glu Cys Val Glu Thr Ile Asn Asn
145                 150                 155                 160

Tyr Thr Cys Lys Cys Asp Pro Gly Phe Ser Gly Leu Lys Cys Glu Gln
                165                 170                 175

Ile Val Asn Cys Thr Ala Leu Glu Ser Pro Glu His Gly Ser Leu Val
            180                 185                 190

Cys Ser His Pro Leu Gly Asn Phe Ser Tyr Asn Ser Ser Cys Ser Ile
        195                 200                 205

Ser Cys Asp Arg Gly Tyr Leu Pro Ser Ser Met Glu Thr Met Gln Cys
    210                 215                 220

Met Ser Ser Gly Glu Trp Ser Ala Pro Ile Pro Ala Cys Asn Val Val
225                 230                 235                 240

Glu Cys Asp Ala Val Thr Asn Pro Ala Asn Gly Phe Val Glu Cys Phe
                245                 250                 255

Gln Asn Pro Gly Ser Phe Pro Trp Asn Thr Thr Cys Thr Phe Asp Cys
            260                 265                 270

Glu Glu Gly Phe Glu Leu Met Gly Ala Gln Ser Leu Gln Cys Thr Ser
        275                 280                 285

Ser Gly Asn Trp Asp Asn Glu Lys Pro Thr Cys Lys Ala Val Thr Cys
    290                 295                 300

Arg Ala Val Arg Gln Pro Gln Asn Gly Ser Val Arg Cys Ser His Ser
305                 310                 315                 320

Pro Ala Gly Glu Phe Thr Phe Lys Ser Ser Cys Asn Phe Thr Cys Glu
                325                 330                 335

Glu Gly Phe Met Leu Gln Gly Pro Ala Gln Val Glu Cys Thr Thr Gln
            340                 345                 350

Gly Gln Trp Thr Gln Gln Ile Pro Val Cys Glu Ala Phe Gln Cys Thr
        355                 360                 365

Ala Leu Ser Asn Pro Glu Arg Gly Tyr Met Asn Cys Leu Pro Ser Ala
    370                 375                 380

Ser Gly Ser Phe Arg Tyr Gly Ser Ser Cys Glu Phe Ser Cys Glu Gln
385                 390                 395                 400

Gly Phe Val Leu Lys Gly Ser Lys Arg Leu Gln Cys Gly Pro Thr Gly
                405                 410                 415

Glu Trp Asp Asn Glu Lys Pro Thr Cys Glu Ala Val Arg Cys Asp Ala
            420                 425                 430

Val His Gln Pro Pro Lys Gly Leu Val Arg Cys Ala His Ser Pro Ile
        435                 440                 445

Gly Glu Phe Thr Tyr Lys Ser Ser Cys Ala Phe Ser Cys Glu Glu Gly
    450                 455                 460

Phe Glu Leu His Gly Ser Thr Gln Leu Glu Cys Thr Ser Gln Gly Gln
465                 470                 475                 480

Trp Thr Glu Glu Val Pro Ser Cys Gln Val Val Lys Cys Ser Ser Leu
                485                 490                 495

Ala Val Pro Gly Lys Ile Asn Met Ser Cys Ser Gly Glu Pro Val Phe
            500                 505                 510

Gly Thr Val Cys Lys Phe Ala Cys Pro Glu Gly Trp Thr Leu Asn Gly
        515                 520                 525

Ser Ala Ala Arg Thr Cys Gly Ala Thr Gly His Trp Ser Gly Leu Leu
    530                 535                 540

Pro Thr Cys Glu Ala Pro Thr Glu Ser Asn Ile Pro Leu Val Ala Gly
545                 550                 555                 560

Leu Ser Ala Ala Gly Leu Ser Leu Leu Thr Leu Ala Pro Phe Leu Leu
                565                 570                 575

Trp Leu Arg Lys Cys Leu Arg Lys Ala Lys Lys Phe Val Pro Ala Ser
            580                 585                 590

Ser Cys Gln Ser Leu Glu Ser Asp Gly Ser Tyr Gln Lys Pro Ser Tyr
        595                 600                 605

Ile Leu
    610

SEQUENCE LISTING

<110>  BIOFTALMIK S.L.

<120>  DIAGNOSTIC METHOD FOR DIAGNOSING INFANT EYE ALLERGY

<130>  P6388EP00

<160>  10

<170>  PatentIn version 3.5

<210>  1
<211>  275
<212>  PRT
<213>  Homo sapiens

<400>  1

Met Leu Ser Leu Leu Leu Leu Ala Leu Pro Val Leu Ala Ser Arg Ala
1               5                   10                  15

Tyr Ala Ala Pro Ala Pro Val Gln Ala Leu Gln Gln Ala Gly Ile Val
            20                  25                  30

Gly Gly Gln Glu Ala Pro Arg Ser Lys Trp Pro Trp Gln Val Ser Leu
        35                  40                  45

Arg Val Arg Asp Arg Tyr Trp Met His Phe Cys Gly Gly Ser Leu Ile
    50                  55                  60

His Pro Gln Trp Val Leu Thr Ala Ala His Cys Leu Gly Pro Asp Val
65                  70                  75                  80

Lys Asp Leu Ala Thr Leu Arg Val Gln Leu Arg Glu Gln His Leu Tyr
                85                  90                  95

Tyr Gln Asp Gln Leu Leu Pro Val Ser Arg Ile Ile Val His Pro Gln
            100                 105                 110

Phe Tyr Ile Ile Gln Thr Gly Ala Asp Ile Ala Leu Leu Glu Leu Glu
        115                 120                 125

Glu Pro Val Asn Ile Ser Ser Arg Val His Thr Val Met Leu Pro Pro
    130                 135                 140

Ala Ser Glu Thr Phe Pro Pro Gly Met Pro Cys Trp Val Thr Gly Trp
145                 150                 155                 160

Gly Asp Val Asp Asn Asp Glu Pro Leu Pro Pro Pro Phe Pro Leu Lys
                165                 170                 175

Gln Val Lys Val Pro Ile Met Glu Asn His Ile Cys Asp Ala Lys Tyr
            180                 185                 190

His Leu Gly Ala Tyr Thr Gly Asp Asp Val Arg Ile Ile Arg Asp Asp
        195                 200                 205

Met Leu Cys Ala Gly Asn Thr Arg Arg Asp Ser Cys Lys Gly Asp Ser
    210                 215                 220

Gly Gly Pro Leu Val Cys Lys Val Asn Gly Thr Trp Leu Gln Ala Gly
225                 230                 235                 240

Val Val Ser Trp Asp Glu Gly Cys Ala Gln Pro Asn Arg Pro Gly Ile
                245                 250                 255

Tyr Thr Arg Val Thr Tyr Tyr Leu Asp Trp Ile His His Tyr Val Pro
            260                 265                 270

Lys Lys Pro
        275

<210>  2
<211>  266
<212>  PRT
<213>  Homo sapiens

<400>  2

Met Leu Asn Leu Leu Leu Leu Ala Leu Pro Val Leu Ala Ser Arg Ala
1               5                   10                  15

Tyr Ala Ala Pro Ala Pro Gly Gln Ala Leu Gln Arg Val Gly Ile Val
            20                  25                  30

Gly Gly Gln Glu Ala Pro Arg Ser Lys Trp Pro Trp Gln Val Ser Leu
        35                  40                  45

Arg Val His Gly Pro Tyr Trp Met His Phe Cys Gly Gly Ser Leu Ile
    50                  55                  60

His Pro Gln Trp Val Leu Thr Ala Ala His Cys Val Gly Pro Val Gln
65                  70                  75                  80

Leu Arg Glu Gln His Leu Tyr Tyr Gln Asp Gln Leu Leu Pro Val Ser
                85                  90                  95

Arg Ile Ile Val His Pro Gln Phe Tyr Thr Ala Gln Ile Gly Ala Asp
            100                 105                 110

Ile Ala Leu Leu Glu Leu Glu Glu Pro Val Asn Val Ser Ser His Val
        115                 120                 125

His Thr Val Thr Leu Pro Pro Ala Ser Glu Thr Phe Pro Pro Gly Met
    130                 135                 140

Pro Cys Trp Val Thr Gly Trp Gly Asp Val Asp Asn Asp Glu Arg Leu
145                 150                 155                 160

Pro Pro Pro Phe Pro Leu Lys Gln Val Lys Val Pro Ile Met Glu Asn
                165                 170                 175

His Ile Cys Asp Ala Lys Tyr His Leu Gly Ala Tyr Thr Gly Asp Asp
            180                 185                 190

Val Arg Ile Val Arg Asp Asp Met Leu Cys Ala Gly Asn Thr Arg Arg
        195                 200                 205

Asp Ser Cys Gln Gly Asp Ser Gly Gly Pro Leu Val Cys Lys Val Asn
    210                 215                 220

Gly Thr Trp Leu Gln Ala Gly Val Val Ser Trp Gly Glu Gly Cys Ala
225                 230                 235                 240

Gln Pro Asn Arg Pro Gly Ile Tyr Thr Arg Val Thr Tyr Tyr Leu Asp
                245                 250                 255

Trp Ile His His Tyr Val Pro Lys Lys Pro
            260                 265

<210>  3
<211>  275
<212>  PRT
<213>  Homo sapiens

<400>  3

Met Leu Asn Leu Leu Leu Leu Ala Leu Pro Val Leu Ala Ser Arg Ala
1               5                   10                  15

Tyr Ala Ala Pro Ala Pro Gly Gln Ala Leu Gln Arg Val Gly Ile Val
            20                  25                  30

Gly Gly Gln Glu Ala Pro Arg Ser Lys Trp Pro Trp Gln Val Ser Leu
        35                  40                  45

Arg Val His Gly Pro Tyr Trp Met His Phe Cys Gly Gly Ser Leu Ile
    50                  55                  60

His Pro Gln Trp Val Leu Thr Ala Ala His Cys Val Gly Pro Asp Val
65                  70                  75                  80

Lys Asp Leu Ala Ala Leu Arg Val Gln Leu Arg Glu Gln His Leu Tyr
                85                  90                  95

Tyr Gln Asp Gln Leu Leu Pro Val Ser Arg Ile Ile Val His Pro Gln
            100                 105                 110

Phe Tyr Thr Ala Gln Ile Gly Ala Asp Ile Ala Leu Leu Glu Leu Glu
        115                 120                 125

Glu Pro Val Asn Val Ser Ser His Val His Thr Val Thr Leu Pro Pro
    130                 135                 140

Ala Ser Glu Thr Phe Pro Pro Gly Met Pro Cys Trp Val Thr Gly Trp
145                 150                 155                 160

Gly Asp Val Asp Asn Asp Glu Arg Leu Pro Pro Pro Phe Pro Leu Lys
                165                 170                 175

Gln Val Lys Val Pro Ile Met Glu Asn His Ile Cys Asp Ala Lys Tyr
            180                 185                 190

His Leu Gly Ala Tyr Thr Gly Asp Asp Val Arg Ile Val Arg Asp Asp
        195                 200                 205

Met Leu Cys Ala Gly Asn Thr Arg Arg Asp Ser Cys Gln Gly Asp Ser
    210                 215                 220

Gly Gly Pro Leu Val Cys Lys Val Asn Gly Thr Trp Leu Gln Ala Gly
225                 230                 235                 240

Val Val Ser Trp Gly Glu Gly Cys Ala Gln Pro Asn Arg Pro Gly Ile
                245                 250                 255

Tyr Thr Arg Val Thr Tyr Tyr Leu Asp Trp Ile His His Tyr Val Pro
            260                 265                 270

Lys Lys Pro
        275

<210>  4
<211>  242
<212>  PRT
<213>  Homo sapiens

<400>  4

Met Leu Leu Leu Ala Pro Gln Met Leu Ser Leu Leu Leu Leu Ala Leu
1               5                   10                  15

Pro Val Leu Ala Ser Pro Ala Tyr Val Ala Pro Ala Pro Gly Gln Ala
            20                  25                  30

Leu Gln Gln Thr Gly Ile Val Gly Gly Gln Glu Ala Pro Arg Ser Lys
        35                  40                  45

Trp Pro Trp Gln Val Ser Leu Arg Val Arg Gly Pro Tyr Trp Met His
    50                  55                  60

Phe Cys Gly Gly Ser Leu Ile His Pro Gln Trp Val Leu Thr Ala Ala
65                  70                  75                  80

His Cys Val Glu Pro Asp Ile Lys Asp Leu Ala Ala Leu Arg Val Gln
                85                  90                  95

Leu Arg Glu Gln His Leu Tyr Tyr Gln Asp Gln Leu Leu Pro Val Ser
            100                 105                 110

Arg Ile Ile Val His Pro Gln Phe Tyr Ile Ile Gln Thr Gly Ala Asp
        115                 120                 125

Ile Ala Leu Leu Glu Leu Glu Glu Pro Val Asn Ile Ser Ser His Ile
    130                 135                 140

His Thr Val Thr Leu Pro Pro Ala Ser Glu Thr Phe Pro Pro Gly Met
145                 150                 155                 160

Pro Cys Trp Val Thr Gly Trp Gly Asp Val Asp Asn Asn Val His Leu
                165                 170                 175

Pro Pro Pro Tyr Pro Leu Lys Glu Val Glu Val Pro Val Val Glu Asn
            180                 185                 190

His Leu Cys Asn Ala Glu Tyr His Thr Gly Leu His Thr Gly His Ser
        195                 200                 205

Phe Gln Ile Val Arg Asp Asp Met Leu Cys Ala Gly Ser Glu Asn His
    210                 215                 220

Asp Ser Cys Gln Gly Asp Ser Gly Gly Pro Leu Val Cys Lys Val Asn
225                 230                 235                 240

Gly Thr

<210>  5
<211>  321
<212>  PRT
<213>  Homo sapiens

<400>  5

Met Ala Leu Gly Ala Cys Gly Leu Leu Leu Leu Leu Ala Val Pro Gly
1               5                   10                  15

Val Ser Leu Arg Thr Leu Gln Pro Gly Cys Gly Arg Pro Gln Val Ser
            20                  25                  30

Asp Ala Gly Gly Arg Ile Val Gly Gly His Ala Ala Pro Ala Gly Ala
        35                  40                  45

Trp Pro Trp Gln Ala Ser Leu Arg Leu Arg Arg Met His Val Cys Gly
    50                  55                  60

Gly Ser Leu Leu Ser Pro Gln Trp Val Leu Thr Ala Ala His Cys Phe
65                  70                  75                  80

Ser Gly Ser Leu Asn Ser Ser Asp Tyr Gln Val His Leu Gly Glu Leu
                85                  90                  95

Glu Ile Thr Leu Ser Pro His Phe Ser Thr Val Arg Gln Ile Ile Leu
            100                 105                 110

His Ser Ser Pro Ser Gly Gln Pro Gly Thr Ser Gly Asp Ile Ala Leu
        115                 120                 125

Val Glu Leu Ser Val Pro Val Thr Leu Ser Ser Arg Ile Leu Pro Val
    130                 135                 140

Cys Leu Pro Glu Ala Ser Asp Asp Phe Cys Pro Gly Ile Arg Cys Trp
145                 150                 155                 160

Val Thr Gly Trp Gly Tyr Thr Arg Glu Gly Glu Pro Leu Pro Pro Pro
                165                 170                 175

Tyr Ser Leu Arg Glu Val Lys Val Ser Val Val Asp Thr Glu Thr Cys
            180                 185                 190

Arg Arg Asp Tyr Pro Gly Pro Gly Gly Ser Ile Leu Gln Pro Asp Met
        195                 200                 205

Leu Cys Ala Arg Gly Pro Gly Asp Ala Cys Gln Asp Asp Ser Gly Gly
    210                 215                 220

Pro Leu Val Cys Gln Val Asn Gly Ala Trp Val Gln Ala Gly Thr Val
225                 230                 235                 240

Ser Trp Gly Glu Gly Cys Gly Arg Pro Asn Arg Pro Gly Val Tyr Thr
                245                 250                 255

Arg Val Pro Ala Tyr Val Asn Trp Ile Arg Arg His Ile Thr Ala Ser
            260                 265                 270

Gly Gly Ser Glu Ser Gly Tyr Pro Arg Leu Pro Leu Leu Ala Gly Leu
        275                 280                 285

Phe Leu Pro Gly Leu Phe Leu Leu Leu Val Ser Cys Val Leu Leu Ala
    290                 295                 300

Lys Cys Leu Leu His Pro Ser Ala Asp Gly Thr Pro Phe Pro Ala Pro
305                 310                 315                 320

Asp

<210>  6
<211>  97
<212>  PRT
<213>  Homo sapiens

<400>  6

Met Lys Val Ser Ala Ala Leu Leu Trp Leu Leu Leu Ile Ala Ala Ala
1               5                   10                  15

Phe Ser Pro Gln Gly Leu Ala Gly Pro Ala Ser Val Pro Thr Thr Cys
            20                  25                  30

Cys Phe Asn Leu Ala Asn Arg Lys Ile Pro Leu Gln Arg Leu Glu Ser
        35                  40                  45

Tyr Arg Arg Ile Thr Ser Gly Lys Cys Pro Gln Lys Ala Val Ile Phe
    50                  55                  60

Lys Thr Lys Leu Ala Lys Asp Ile Cys Ala Asp Pro Lys Lys Lys Trp
65                  70                  75                  80

Val Gln Asp Ser Met Lys Tyr Leu Asp Gln Lys Ser Pro Thr Pro Lys
                85                  90                  95

Pro

<210>  7
<211>  222
<212>  PRT
<213>  Homo sapiens

<400>  7
   Met Lys Leu Pro Leu Leu Leu Ala Leu Leu Phe Gly Ala Val Ser Ala
   1               5                   10                  15
   Leu His Leu Arg Ser Glu Thr Ser Thr Phe Glu Thr Pro Leu Gly Ala
               20                  25                  30
   Lys Thr Leu Pro Glu Asp Glu Glu Thr Pro Glu Gln Glu Met Glu Glu
   35 40 45
   Thr Pro Cys Arg Glu Leu Glu Glu Glu Glu Glu Trp Gly Ser Gly Ser
   50 55 60
   Glu Asp Ala Ser Lys Lys Asp Gly Ala Val Glu Ser Ile Ser Val Pro
   65 70 75 80
   Asp Met Val Asp Lys Asn Leu Thr Cys Pro Glu Glu Glu Asp Thr Val
   85 90 95
   Lys Val Val Gly Ile Pro Gly Cys Gln Thr Cys Arg Tyr Leu Leu Val
   100 105 110
   Arg Ser Leu Gln Thr Phe Ser Gln Ala Trp Phe Thr Cys Arg Arg Cys
   115 120 125
   Tyr Arg Gly Asn Leu Val Ser Ile His Asn Phe Asn Ile Asn Tyr Arg
   130 135 140
   Ile Gln Cys Ser Val Ser Ala Leu Asn Gln Gly Gln Val Trp Ile Gly
   145 150 155 160
   Gly Arg Ile Thr Gly Ser Gly Arg Cys Arg Arg Phe Gln Trp Val Asp
   165 170 175
   Gly Ser Arg Trp Asn Phe Ala Tyr Trp Ala Ala His Gln Pro Trp Ser
   180 185 190
   Arg Gly Gly His Cys Val Ala Leu Cys Thr Arg Gly Gly His Trp Arg
   195 200 205
   Arg Ala His Cys Leu Arg Arg Leu Pro Phe Ile Cys Ser Tyr
   210 215 220

<210>  8
<211>  160
<212>  PRT
<213>  Homo sapiens

<400>  8

Met Val Pro Lys Leu Phe Thr Ser Gln Ile Cys Leu Leu Leu Leu Leu
1               5                   10                  15

Gly Leu Met Gly Val Glu Gly Ser Leu His Ala Arg Pro Pro Gln Phe
            20                  25                  30

Thr Arg Ala Gln Trp Phe Ala Ile Gln His Ile Ser Leu Asn Pro Pro
        35                  40                  45

Arg Cys Thr Ile Ala Met Arg Ala Ile Asn Asn Tyr Arg Trp Arg Cys
    50                  55                  60

Lys Asn Gln Asn Thr Phe Leu Arg Thr Thr Phe Ala Asn Val Val Asn
65                  70                  75                  80

Val Cys Gly Asn Gln Ser Ile Arg Cys Pro His Asn Arg Thr Leu Asn
                85                  90                  95

Asn Cys His Arg Ser Arg Phe Arg Val Pro Leu Leu His Cys Asp Leu
            100                 105                 110

Ile Asn Pro Gly Ala Gln Asn Ile Ser Asn Cys Thr Tyr Ala Asp Arg
        115                 120                 125

Pro Gly Arg Arg Phe Tyr Val Val Ala Cys Asp Asn Arg Asp Pro Arg
    130                 135                 140

Asp Ser Pro Arg Tyr Pro Val Val Pro Val His Leu Asp Thr Thr Ile
145                 150                 155                 160

<210>  9
<211>  161
<212>  PRT
<213>  Homo sapiens

<400>  9

Met Val Pro Lys Leu Phe Thr Ser Gln Ile Cys Leu Leu Leu Leu Leu
1               5                   10                  15

Gly Leu Leu Ala Val Glu Gly Ser Leu His Val Lys Pro Pro Gln Phe
            20                  25                  30

Thr Trp Ala Gln Trp Phe Glu Thr Gln His Ile Asn Met Thr Ser Gln
        35                  40                  45

Gln Cys Thr Asn Ala Met Gln Val Ile Asn Asn Tyr Gln Arg Arg Cys
    50                  55                  60

Lys Asn Gln Asn Thr Phe Leu Leu Thr Thr Phe Ala Asn Val Val Asn
65                  70                  75                  80

Val Cys Gly Asn Pro Asn Met Thr Cys Pro Ser Asn Lys Thr Arg Lys
                85                  90                  95

Asn Cys His His Ser Gly Ser Gln Val Pro Leu Ile His Cys Asn Leu
            100                 105                 110

Thr Thr Pro Ser Pro Gln Asn Ile Ser Asn Cys Arg Tyr Ala Gln Thr
        115                 120                 125

Pro Ala Asn Met Phe Tyr Ile Val Ala Cys Asp Asn Arg Asp Gln Arg
    130                 135                 140

Arg Asp Pro Pro Gln Tyr Pro Val Val Pro Val His Leu Asp Arg Ile
145                 150                 155                 160

Ile

<210>  10
<211>  610
<212>  PRT
<213>  Homo sapiens

<400>  10

Met Ile Ala Ser Gln Phe Leu Ser Ala Leu Thr Leu Val Leu Leu Ile
1               5                   10                  15

Lys Glu Ser Gly Ala Trp Ser Tyr Asn Thr Ser Thr Glu Ala Met Thr
            20                  25                  30

Tyr Asp Glu Ala Ser Ala Tyr Cys Gln Gln Arg Tyr Thr His Leu Val
        35                  40                  45

Ala Ile Gln Asn Lys Glu Glu Ile Glu Tyr Leu Asn Ser Ile Leu Ser
    50                  55                  60

Tyr Ser Pro Ser Tyr Tyr Trp Ile Gly Ile Arg Lys Val Asn Asn Val
65                  70                  75                  80

Trp Val Trp Val Gly Thr Gln Lys Pro Leu Thr Glu Glu Ala Lys Asn
                85                  90                  95

Trp Ala Pro Gly Glu Pro Asn Asn Arg Gln Lys Asp Glu Asp Cys Val
            100                 105                 110

Glu Ile Tyr Ile Lys Arg Glu Lys Asp Val Gly Met Trp Asn Asp Glu
        115                 120                 125

Arg Cys Ser Lys Lys Lys Leu Ala Leu Cys Tyr Thr Ala Ala Cys Thr
    130                 135                 140

Asn Thr Ser Cys Ser Gly His Gly Glu Cys Val Glu Thr Ile Asn Asn
145                 150                 155                 160

Tyr Thr Cys Lys Cys Asp Pro Gly Phe Ser Gly Leu Lys Cys Glu Gln
                165                 170                 175

Ile Val Asn Cys Thr Ala Leu Glu Ser Pro Glu His Gly Ser Leu Val
            180                 185                 190

Cys Ser His Pro Leu Gly Asn Phe Ser Tyr Asn Ser Ser Cys Ser Ile
        195                 200                 205

Ser Cys Asp Arg Gly Tyr Leu Pro Ser Ser Met Glu Thr Met Gln Cys
    210                 215                 220

Met Ser Ser Gly Glu Trp Ser Ala Pro Ile Pro Ala Cys Asn Val Val
225                 230                 235                 240

Glu Cys Asp Ala Val Thr Asn Pro Ala Asn Gly Phe Val Glu Cys Phe
                245                 250                 255

Gln Asn Pro Gly Ser Phe Pro Trp Asn Thr Thr Cys Thr Phe Asp Cys
            260                 265                 270

Glu Glu Gly Phe Glu Leu Met Gly Ala Gln Ser Leu Gln Cys Thr Ser
        275                 280                 285

Ser Gly Asn Trp Asp Asn Glu Lys Pro Thr Cys Lys Ala Val Thr Cys
    290                 295                 300

Arg Ala Val Arg Gln Pro Gln Asn Gly Ser Val Arg Cys Ser His Ser
305                 310                 315                 320

Pro Ala Gly Glu Phe Thr Phe Lys Ser Ser Cys Asn Phe Thr Cys Glu
                325                 330                 335

Glu Gly Phe Met Leu Gln Gly Pro Ala Gln Val Glu Cys Thr Thr Gln
            340                 345                 350

Gly Gln Trp Thr Gln Gln Ile Pro Val Cys Glu Ala Phe Gln Cys Thr
        355                 360                 365

Ala Leu Ser Asn Pro Glu Arg Gly Tyr Met Asn Cys Leu Pro Ser Ala
    370                 375                 380

Ser Gly Ser Phe Arg Tyr Gly Ser Ser Cys Glu Phe Ser Cys Glu Gln
385                 390                 395                 400

Gly Phe Val Leu Lys Gly Ser Lys Arg Leu Gln Cys Gly Pro Thr Gly
                405                 410                 415

Glu Trp Asp Asn Glu Lys Pro Thr Cys Glu Ala Val Arg Cys Asp Ala
            420                 425                 430

Val His Gln Pro Pro Lys Gly Leu Val Arg Cys Ala His Ser Pro Ile
        435                 440                 445

Gly Glu Phe Thr Tyr Lys Ser Ser Cys Ala Phe Ser Cys Glu Glu Gly
    450                 455                 460

Phe Glu Leu His Gly Ser Thr Gln Leu Glu Cys Thr Ser Gln Gly Gln
465                 470                 475                 480

Trp Thr Glu Glu Val Pro Ser Cys Gln Val Val Lys Cys Ser Ser Leu
                485                 490                 495

Ala Val Pro Gly Lys Ile Asn Met Ser Cys Ser Gly Glu Pro Val Phe
            500                 505                 510

Gly Thr Val Cys Lys Phe Ala Cys Pro Glu Gly Trp Thr Leu Asn Gly
        515                 520                 525

Ser Ala Ala Arg Thr Cys Gly Ala Thr Gly His Trp Ser Gly Leu Leu
    530                 535                 540

Pro Thr Cys Glu Ala Pro Thr Glu Ser Asn Ile Pro Leu Val Ala Gly
545                 550                 555                 560

Leu Ser Ala Ala Gly Leu Ser Leu Leu Thr Leu Ala Pro Phe Leu Leu
                565                 570                 575

Trp Leu Arg Lys Cys Leu Arg Lys Ala Lys Lys Phe Val Pro Ala Ser
            580                 585                 590

Ser Cys Gln Ser Leu Glu Ser Asp Gly Ser Tyr Gln Lys Pro Ser Tyr
        595                 600                 605

Ile Leu
    610